# EUROPEAN PATENT APPLICATION

(11) **EP 1 295 599 A1**
(43) Date of publication of application: **26.03.2003**
(21) Application number: 01122730.3
(22) Date of filing: 21.09.2001
(51) Int. Cl.: A61K 31/495, A61K 31/445, A61P 17/04

(54) **Method for the treatment of prevention of atopic dermatitis**

(71) Applicant: Boehringer Ingelheim International GmbH, 55216 Ingelheim am Rhein (DE)
(72) Inventor: Komune, Kunihiko, Kawanishi, Obana 2-7-3-1102 (JP); Ohmura, Tsuyoshi, Dr., Osaka 593-0105 (JP); Satoh, Hisashi, Dr., Ibaraki-shi, Osaka 567-0072 (JP)

(57) **Abstract**

The invention relates to a method for the treatment or prevention of atopic dermatitis, which comprises the administration of an effective amount of an NK-1 receptor antagonist to a patient in need of such treatment, wherein said NK1 receptor antagonist is effective in inhibiting the substance P (SP-)induced scratching in mice.

## Description

The invention relates to a method for the treatment or prevention of atopic dermatitis, which comprises the administration of an effective amount of an NK-1 receptor antagonist to a patient in need of such treatment, wherein said NK1 receptor antagonist is effective in inhibiting the substance P (SP-)induced scratching in mice.

### Background of the Invention

Itch is a sensation that provokes a desire to scratch. It is the most common symptom of cutaneous disease (e.g., atopic dermatitis and contact dermatitis) but its underlying mechanisms are far from being understood. This sensation is produced experimentally by several endogenous substances such as histamine, substance P (SP), vasoactive intestinal peptide and neurotensin.

SP is one of the most potent pruitogenic endogenous peptides (Hagermark O; Peripheral and central mediators of itch. Skin Pharmacol 1992;5:1-8) and may be involved in some pruritic disease (Farber EM, et al., Symmetry, and Psoriasis: Possible Role of Neuropeptides. J Am Acad Dermatol 1986;14:305-11). Basic peptides generally degranulate mast cells and SP produces the degranulation of mast cells (Devillier P, et al., Role of the N-terminal arginine in the histamine-releasing activity of substance P, bradykinin and related peptides. Eur J Pharmacol 1989;168:53-60; Ebertz JM, et al., J Invest Dermatol 1987; 88: 682-5 Substance P-induced histamine release in human cutaneous mast cells.) and itch induced by SP is thought to be mediated by histamine release from mast cells. Kuraishi et al (Eur J Pharmacol 1995; 275: 229-33 Scratching behavior induced by pruritogenic but not algesiogenic agents in mice.) indicated that SP elicited an apparent scratching in ddY mice and it suggests that SP must play an important role in the scratching. Inagaki et al. (Eur J Pharmacol 2000;400:73-9 Evaluation of anti-scratch properties of oxatomide and epinastine in mice.) also reported that epinastine showed a significant effect on SP-induced scratching.

Accordingly, there is a high need for new, safe and effective treatment of atopic dermatitis. Surprisingly it has been found that certain NK1 receptor antagonists, which are effective in reducing the SP-induced scratching in mice, can be used to treat or prevent atopic dermatitis.

### Detailed Description of the Invention

Therefore the present invention relates to a method for the treatment or prevention atopic dermatitis of, which comprises the administration of an effective amount of an NK-1 receptor antagonist to a patient in need of such treatment, wherein said NK1 receptor antagonist is effective in inhibiting the SP-induced scratching in mice.

Another aspect of the present invention resides in the use of an NK-1 receptor antagonist, which is effective in inhibiting the SP-induced scratching in mice for the preparation of a medication for the treatment or prevention of atopic dermatitis.

The exceptional pharmacology of the class of NK-1 receptor antagonists of use in the present invention enables the treatment or prevention of atopic dermatitis, without the need for concomitant therapy using corticosteroides.

Furthermore, the exceptional pharmacology of the class of NK-1 receptor antagonists of use in the present invention results in a rapid onset of action.

In a further aspect of the present invention, there is provided a pharmaceutical composition for the treatment of atopic dermatitis, which comprises a long acting, NK-1 receptor antagonist, is effective in inhibiting the SP-induced scratching in mice (as hereinafter defined), together with a pharmaceutically acceptable carrier or excipient.

There exists a patient population in whom the condition of atopic dermatitis is inadequately treated with existing corticosteroide therapy. Furthermore, some patients may be adversely affected by the side-effects of existing therapies.

Therefore, the invention preferably relates to a method of treatment or prevention of atopic dermatitis, which comprises the administration of an effective amount of an NK-1 receptor antagonist without concomitant therapy with corticosteroides.

As used herein, the term "non-responsive" in relation to atopic dermatitis means patients who have not had a reasonable clinical response (e.g. a 50% reduction in Hamilton Dermatitis Scale (HAM-D) from a patient's baseline score after treatment with one or more clinical courses of conventional anti-dermatitis medication.

Accordingly, the invention preferably relates to a method of treatment or prevention of atopic dermatitis, which comprises the administration of an effective amount of an NK-1 receptor antagonist, wherein said NK1 receptor antagonist is effective in reducing the SP-induced scratching in mice for at least 50 %, in particular for at least 60 %.

In particular the present invention provides a method for the treatment or prevention of atopic dermatitis, wherein said NK-1 receptor antagonist, is administered orally or topically.

NK-1 receptor antagonists are described in published European Patent Specification Nos. 0 360 390, 0 394 989, 0 429 366, 0 443 132, 0 482 539, 0 512 901, 0 512 902, 0 514 273, 0 514 275, 0 517 589, 0 520 555, 0 522 808, 0 528 495, 0 532 456, 0 533 280, 0 536 817, 0 545 478, 0 577 394, 0 590 152, 0 599 538, 0 610 793, 0 634 402, 0 686 629, 0 693 489, 0 694 535, 0 699 655, 0 699 674, 0 707 006, 0 708 101, 0 714 891, 0 723 959, 0 733 632 and 0 776 893; and in International Patent Specification Nos. 90/05525, 90/05729, 91/09844, 91/18899, 92/01688, 92/06079, 92/12151, 92/15585, 92117449, 92/20661, 92/20676, 92/21677, 93/00330, 93/00331, 93/01159, 93/01165, 93/01169, 93/01170, 93/06099, 93/09116, 93/10073, 93/14113, 93/18023, 93/19064, 93/21155, 9321181, 93/23380, 93/24465, 94/01402, 94/02461, 94/03429, 94/03445, 94/04494, 94/04496, 94/05625, 94/07843, 94/10165, 94/10167, 94/10168, 94/10170, 94/11368, 94/13639, 94/13663, 94/14767, 94/15903, 94/19320, 94/19323, 94/20500, 94/26735, 94/26740, 94/29309, 95/02595, 95/04040, 95/04042, 95/06645, 95/07886, 95/07908, 95/08549, 95/11880, 95/14017, 95/15311, 95/16679, 95/17382, 95/18124, 95/18129, 95/19344, 95/20575, 95121819, 96122525, 95123798, 95/26338, 95/28418, 95/30674, 95/30687, 96/05193, 96/05203, 96/06094, 96/07649, 96/10562, 96/16939, 96/18643, 96/20197, 96/21661, 96/29304, 96/29317, 96129326, 96/29328, 96/31214, 96/32386, 96/37489, 97/01553, 97/01554, 97/03066, 97/08144, 97/14671, 97/17362, 97/18206, 97/19084, 97/19942 97/21702; 97/32865, and 99/62893and in British Patent Specification Nos. 2 266 529, 2 268 931, 2 269 170, 2 269 590, 2 271 774, 2 292 144, 2 293 168, 2 293 169, and 2 302 689.

Preferred are long-acting NK1 receptor antagonists, in particular those, which are a phenyl-glycineamides having a structural element of formula (1) 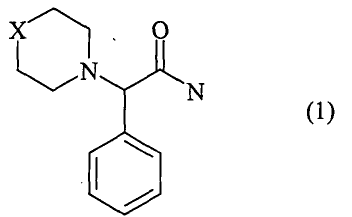 wherein X represents N or CH,
in particular, wherein the structural element of formula (1) consists essentially of the (S)-enantiomer, which are described in the International Patent Applications WO 96/32386, WO 97/32865, and WO 99/62893

Preferred are those NK-1 receptor antagonists which are phenyl-glycineamides of formula (I) 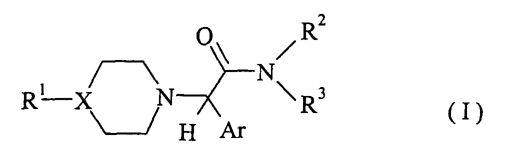 or a pharmaceutically acceptable salts thereof,
wherein
- R¹: represents
(a) a C₃-C₈-cycloalkyl-C₁-C₆-alkyl group, in the event that X is a nitrogen atom; or
(b) an amino group of formula NR⁴R⁵, in the event that X is CH group;
- R²: represents phenyl-C₁-C₄-alkyl, wherein the phenyl group may be substituted by 1 to 3 substituent selected from the group consisting of halogen, hydroxy, C₁-C₄-alkyl, C₁-C₄-alkoxy, C₁-C₄-fluoroalkyl, C₁-C₄-fluoroalkoxy;
- R³: represents hydrogen, C₁-C₄-alkyl, C₃-C₈-cycloalkyl, CH₂COOH, -CH₂C(O)NH₂, -OH or phenyl-C₁-C₄-alkyl;
- R⁴: represents a 3-hydroxypropyl, 1,3-dihydroxyprop-2-yl or C₃-C₆-cycloalkylmethyl group;
- R⁵: represents hydrogen, C₁-C₆-alkyl, ω-hydroxy-C₂-C₄-alkyl, 1,3-dihydroxyprop-2-yl or C₃-C₆-cycloalkylmethyl group; and
- Ar: represents an unsubstituted phenyl group or a phenyl group which is substituted by 1 to 5 substituents selected from the group consisting of halogen, hydroxy, C₁-C₄-alkyl, C₁-C₄-alkoxy, C₁-C₄-fluoroalkyl, C₁-C₄-fluoroalkoxy and -OCH₂O-,
in particular wherein -NR²R³ is a group of formula

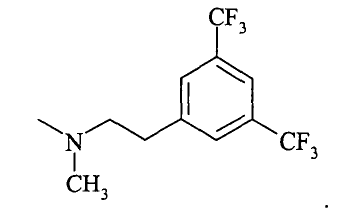

Most preferred are said NK-1 receptor antagonists selected from the formulae (Ia) to (Id):

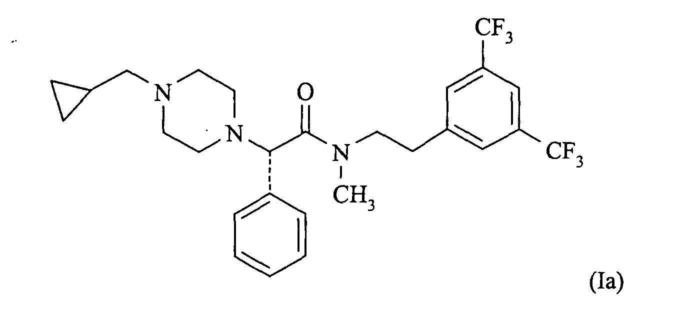

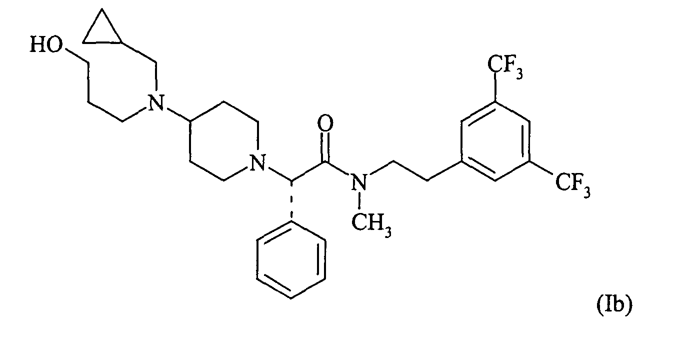

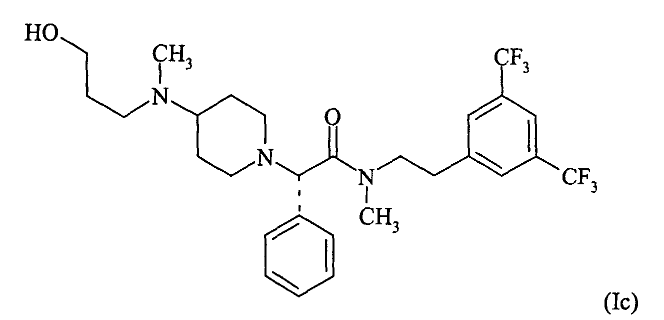

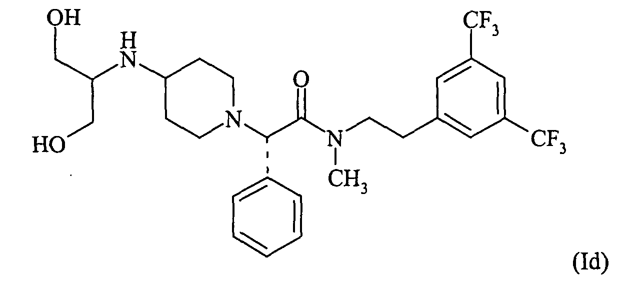

Most preferred are the compounds of formulae Ia and Id.

Full descriptions of the preparation of the NK-1 receptor antagonists which may be employed in the present invention may be found in the references cited herein.

Suitable pharmaceutically acceptable salts of the NK-1 receptor antagonists of use in the present invention include acid addition salts which may, for example, be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid, phosphoric acid or sulphuric acid. Salts of amine groups may also comprise the quaternary ammonium salts in which the amino nitrogen atom carries an alkyl, alkenyl, alkynyl or aralkyl group. Where the compound carries an acidic group, for example a carboxylic acid group, the present invention also contemplates salts thereof, preferably non-toxic pharmaceutically acceptable salts thereof, such as the sodium, potassium and calcium salts thereof.

Preferably the compositions containing an NK-1 receptor antagonist of use according to the present invention are in unit dosage forms such as tablets, pills, capsules, wafers and the like. Additionally, the NK-1 receptor antagonists of use according to the present invention may be presented as granules or powders for extemporaneous formulation as volume defined solutions or suspensions. Alternatively, the NK-1 receptor antagonists of use according to the present invention may be presented in ready-prepared volume defined solutions or suspensions. Preferred forms are tablets and capsules.

For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

Compositions of the present invention may also be administered via the buccal cavity using conventional technology, for example, absorption wafers.

Compositions in the form of tablets, pills, capsules or wafers for oral administration are particularly preferred.

Preferred compositions for topical administrations are creams, lotions, milks, gels and ointments. As a rule such compositions for topical administration are emulsions containing 5 to 80, preferably 10 to 50 % by weight of an oil phase consisting essentially of an oil, wax and/or fat, and one ore more additional adjuvants selected from the group consisting of lipophilic or hydrophylic gelling agents, solvents, fillers, conservation agents antioxidants, perfumes, colorants, dispersing agents and penetration enhancing agents such as DMSO or ethanol.

The following non-limiting examples of recipes illustrate compositions for topical applications according to the invention.

| Topical Formulation A | |
|---|---|
| Component | Amount in g |
| NK-1 receptor antagonist of formula (I) | 0.5 to 15 |
| Emulsifying Wax | 30 |
| Liquid paraffin | 20 |
| White soft paraffin | to 100 |

| Topical Formulation B | |
|---|---|
| Component | Amount in g |
| NK-1 receptor antagonist of formula (I) | 0.5 to 15 |
| Glycerol stearate | 2.0 |
| Polysorbate 60 (e.g. Tween 60 available from ICI) | 1.0 |
| Triethanolamine | 0.7 |
| Carbomer | 0.4 |
| Liquid fraction of shea butter | 12.0 |
| Perhydrosqualen | 12.0 |
| Antioxidant | 0.05 |
| Conserving agent | 0.3 |
| Water | to 100 |

A minimum dosage level for the NK-1 receptor antagonist is about 1 mg per day, preferably about 5 mg per day and especially about 10 mg per day. A maximum dosage level for the NK-1 receptor antagonist is about 1500 mg per day, preferably about 1000 mg per day and especially about 500 mg per day. The compounds are administered once or twice a day.

It will be appreciated that the amount of the NK-1 receptor antagonist required for use in the treatment or prevention of atopic dermatitis will vary not only with the particular compounds or compositions selected but also with the route of administration, the nature of the condition being treated, and the age and condition of the patient, and will ultimately be at the discretion of the patient's physician or pharmacist.

### Brief Description of the Drawings

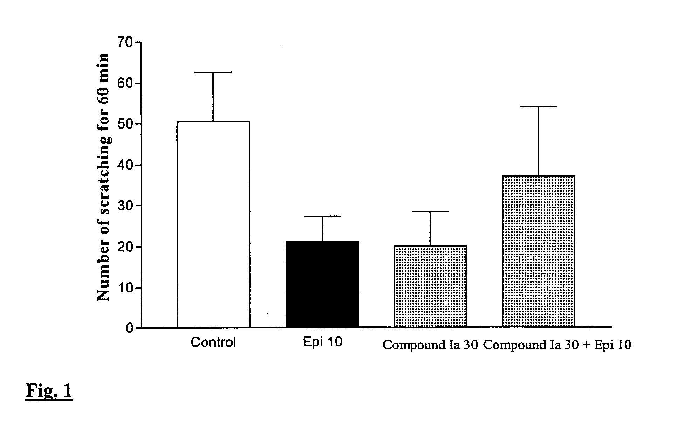

Figure 1 shows the effects of epinastine and Compound Ia on intradermal substance P (150 µg/site) on scratching behavior. The ddY mice were given the oral administration of distilled water (Control), epinastine 10 mg/kg (Epi 10), Compound Ia 30 mg/kg, (Compound Ia 30) and Compound Ia 30 mg/kg + epinastine 10 mg/kg(Compound Ia 30 + Epi 10). Values show mean±SE.

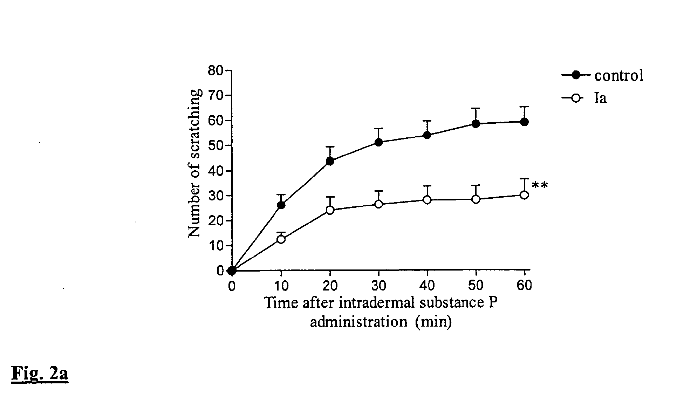

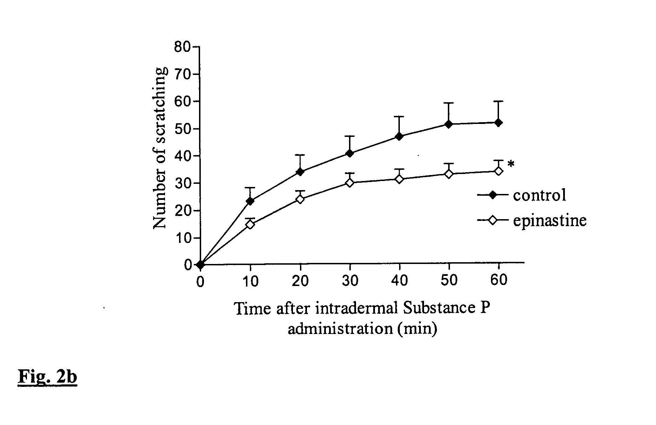

Figures 2a and 2b show the effects of Compound Ia (Fig. 2a) and epinastine (Fig. 2b) on intradermal substance P (150 µg/site) on accumulated scratching behavior, which is monitored for 1 hour. The ddY mice were given the oral administration of distilled water (control), epinastine 10 mg/kg (epinastine) and Compound Ia 30 mg/kg, (Ia) Values show mean±SE.

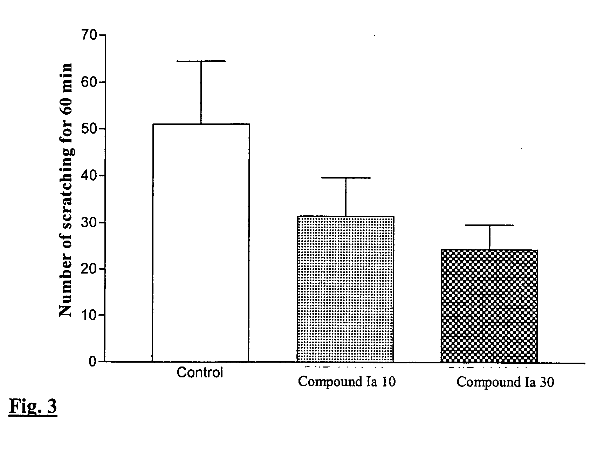

Figure 3 shows the effects of Compound Ia on intradermal substance P (150 µg/site) on scratching behavior. The ddY mice were given the oral administration of distilled water (Control), Compound Ia 10 mg/kg, (Compound Ia 10) and Compound Ia 30 mg/kg, (Compound Ia 30). Values show mean±SE.

Procedures by way of example for preparing the compounds according to the invention will be described in more detail hereinafter. The Examples which follow serve solely as a detailed illustration without restricting the subject matter of the invention.

Three compounds of use in the present invention (Ib to Id) which are described in German Patent Application DE 100 51 320.4 (filed October 17, 2000), the complete disclosure of which is hereby incorporated by reference may be prepared according to the following methods:

### PREPARATION Ib

### (S)-N-[2-(3,5-Bis-trifluormethyl-phenyl)-ethyl]-2-{4-[cyclopropylmethyl-(3-hydroxypropyl)-amino]-piperidin-1-yl}-N-methyl-2-phenyl-acetamid

a) 16.5 g of 3-aminopropanol and 41.7 g of 1-benzyl-4-piperidone are dissolved in 350 ml of methylene chloride and 56 g of sodium triacetoxy-borohydride are slowly added at about 10 °C. The mixture is stirred overnight at ambient temperature, then acidified with dilute hydrochloric acid while cooling and then made alkaline with conc. sodium hydroxide solution. The organic phase is separated off, the aqueous phase is washed once more with 150 ml of methylene chloride. The combined organic phases are dried over sodium sulphate and the solvent is eliminated *in vacuo*. 32 g of 1-benzyl-4-(3-hydroxy-propylamino)-piperidine are obtained as a yellow oil, which is used without further purification in the next reaction step.
b) 13.4 g of 1-benzyl-4-(3-hydroxy-propylamino)-piperidine from the previous reaction are dissolved together with 3.8 g of cyclopropane carboxaldehyde in 250 ml of methanol and at 0 °C combined with 5.1 g of sodium cyanoborohydride. The mixture is stirred overnight at ambient temperature, then acidified with dilute hydrochloric acid while cooling and concentrated by evaporation in vacuo. The mixture is then made alkaline with conc. sodium hydroxide solution and extracted three times with 40 ml of methylene chloride. The combined organic phases are dried over sodium sulphate, filtered and the solvent is eliminated *in vacuo*. The residue is filtered with ethyl acetate / methanol / conc. ammonia solution 20:80:1 over silica gel. After the removal of the solvent, 10.2 g of 1-benzyl-4-[cyclopropylmethyl-(3-hydroxy-propyl)-amino]-piperidine are obtained as a yellow oil.
c) 10.2 g of 1-benzyl-4-[cyclopropylmethyl-(3-hydroxy-propyl)-amino]-piperidine are combined with 2 g of 20 % palladium/charcoal in 100 ml of methanol and hydrogenated at 60 °C for 4 h under 5 bars of hydrogen. The catalyst is separated off, the solvent is eliminated *in vacuo* and 7.3 g of 4-[cyclopropylmethyl-(3-hydroxypropyl)-amino]-piperidine are obtained as a yellow oil.
d) 4.7 g of 4-[cyclopropylmethyl-(3-hydroxy-propyl)-amino]-piperidine are stirred together with 9.6 g of (R)-N-[2-(3,5-bis-trifluoromethyl-phenyl)-ethyl]-2-methanesulphonyloxy-N-methyl-2-phenyl-acetamide (prepared from D-(-)-mandelic acid) and 3.4 ml of triethylamine in 200 ml of acetone for four hours at 65 °C. The mixture is concentrated by evaporation in vacuo, combined with 100 ml of saturated sodium hydrogen carbonate solution and extracted with ethyl acetate. The combined organic fractions are dried over sodium sulphate and the solvent is eliminated *in vacuo*. The residue is chromatographed with methylene chloride / methanol 1:1 over silica gel. The fractions found to be uniform by TLC are collected and the solvents are eliminated *in vacuo*. 5.5 g of (S)-N-[2-(3.5-bis-trifluoromethyl-phenyl)-ethyl]-2-{4-[cyclopropylmethyl-(3 -hydroxy-propyl)-amino] -piperidin- 1-yl}-N-methyl-2-phenylacetamide are obtained as a brownish-yellow oil.

### PREPARATION Ic

### (S)-N-[2-(3,5-Bis-trifluormethyl-phenyl)-ethyl]-2-{4-[(3-hydroxy-propyl)-methyl-amino]-piperidin-1-yl}-N-methyl-2-phenyl-acetamide

a) 33 g of 1-benzyl-4-piperidone and 15 g of 3-aminopropanol are combined with a catalytic amount of p-toluenesulphonic acid in 300 ml of toluene and refluxed using a water separator until the calculated amount of water has been separated off. Then the toluene is distilled off, the residue is dissolved in 250 ml of alcohol and cooled to about 5 °C. A total of 6.6 g of sodium borohydride are added batchwise with stirring and stirred for 30 hours at ambient temperature. 50 ml of acetone are added, the mixture is stirred for about another half hour and the solvent is then eliminated *in vacuo*. The residue is combined with 100 ml of water and extracted twice with 150 ml of methylene chloride. The combined organic phases are dried. The mixture is filtered, the, the solvent is eliminated *in vacuo*, the residue is taken up in 80 ml of alcohol, combined with 40 ml of 32% hydrochloric acid, diluted with acetone and stirred for about one hour. The crystals precipitated are suction filtered and dried. 1-benzyl-4-(3-hydroxypropylamino)-piperidine is obtained as the dihydrochloride.
b) The base is liberated from 47.4 g of 1-benzyl-4-(3-hydroxypropylamino)-piperidine-dihydrochloride, combined with 63 ml of 85% formic acid and 22 ml of 37% formaldehyde solution and stirred for two hours at about 90 ― 100 °C. The mixture is left to cool, 37 ml of formic acid and 11 ml of formaldehyde solution are added and stirred for another hour at about 100 ― 110 °C. The mixture is left to cool, combined with 150 ml of methanol, made alkaline with about 270 ml of 32% sodium hydroxide solution while cooling and stirred for about another 30 minutes at 40 ― 50 °C and the methanol is then distilled off. The residue is extracted with twice 100 ml of methylene chloride, the combined methylene chloride phases are dried, filtered and the solvent is eliminated *in vacuo*. The residue is taken up in 80 ml of ethanol, acidified with 34 ml of 32% hydrochloric acid, combined with 100 ml of acetone and stirred . As soon as crystals are precipitated, further acetone is added. The precipitate is suction filtered, washed with acetone and dried. 42.8 g of 1-benzyl-4-[-(3-hydroxypropyl)-methylamino]-piperidine-dihydrochloride are obtained as a solid.
c) 42.8 g of 1-benzyl-4-[(3-hydroxypropyl)-methylamino]-piperidine-dihydrochloride are dissolved in 450 ml of methaol, combined with 5 g of 5% palladium/charcoal and hydrogenated at about 50 °C with hydrogen at a pressure of 4 ― 5 bar. The catalyst is filtered off, the methanol is distilled off and the residue is stirred into acetone. Ether is added, the mixture is left to stand for about two hours and then the crystals are suction filtered. 28.7 g of 4-[-(3-hydroxypropyl)-methylamino]-piperidine-dihydrochloride are obtained as a solid.
d) 9 g of 4-[-(3-hydroxypropyl)-methylamino]-piperidine-dihydrochloride are dissolved in 125 ml of DMF together with 14.5 g of N-[2-(3,5-bis-trifluoromethyl-phenyl)-ethyl]-2-methanesulphonyloxy-N-methyl-2-phenyl-acetamide (prepared analogously to the method described in WO 99/62893 ), combined with 20.5 g of potassium carbonate and stirred for about four hours at 80 ― 90 °C. After cooling the mixture is poured onto ice, extracted twice with 150 ml of ethyl acetate, the combined organic phases are washed twice with water and dried. The drying agent is filtered off, the solvent is eliminated in vacuo and the residue is chromatographed with methylene chloride / methanol / conc. ammonia solution 95:5:0.5 through silica gel. The fractions found to be uniform by TLC are combined and the solvent is eliminated in vacuo. The residue of 9.5 g is taken up in methanol and combined with 3.4 g of fumaric acid. Then the methanol is distilled off until only a small residue remains, acetone is added and the mixture is stirred for about 30 minutes. The crystals precipitated are suction filtered, washed with acetone and ether and dried. 9 g of N-2- N-[2-(3,5-bis-trifluoromethyl-phenyl)-ethyl]-2-{4-[(3-hydroxy-propyl)-methyl-amino]-piperidin- 1-yl}-N-methyl-2-phenyl-acetamide are obtained as the colourless sesquifumarate, m.p. 139 ― 144 °C.

### PREPARATION Id

### (S)-N-[2-(3,5-Bis-trifluormethyl-phenyl)-ethyl]-2-[4-(2-hydroxy-1-hydroxymethylethylamino)-piperidin-1-yl]-N-methyl-2-phenylacetamid

a) 2.75 g of 2-aminopropane-1,3-diol and 5.9 g of 1-benzyl-4-piperidone are dissolved in 60 ml of methylene chloride and a total of 9.9 g of sodium triacetoxyborohydride are added batchwise while cooling with ice. The mixture is left to stand overnight at ambient temperature. 60 ml of methylene chloride and some water are added, then conc. hydrochloric acid is added while cooling with ice until an acidic reaction is obtained. The mixture is stirred for about another 15 min. while cooling and then made significantly alkaline with 4 N sodium hydroxide solution. The aqueous phase is separated off, the organic phase is washed with a very little water, dried over sodium sulphate and the solvent is eliminated *in vacuo*. 8 g of substance are obtained which are chromatographed with methylene chloride / methanol 8:2 over 150 g of silica gel. The fractions found to be uniform by TLC are combined and the solvent is eliminated *in vacuo*. 7.3 g of 1-benzyl-4-(1,3-dihydroxyprop-2-ylamino)-piperidine are obtained.
b) 34.5 g of 1-benzyl-4-(1,3-dihydroxyprop-2-ylamino)-piperidine are dissolved in 400 ml of methanol, combined with 3.4 g of 20% palladium/charcoal and hydrogenated with hydrogen at 24 - 28 °C at 2.2 bar. Then the catalyst is filtered off and the solvent is eliminated *in vacuo*. 22.7 g of4-(1,3-dihydroxyprop-2-ylamino)-piperidine are obtained as an oil which is used without further purification for the next reaction.
c) 9 g of 4-(1,3-dihydroxyprop-2-ylamino)-piperidine are reacted with 22.7 g of N-[2-(3,5-bis-trifluoromethyl-phenyl)-ethyl]-2-methanesulphonyloxy-N-methyl-2-phenylacetamide in 110 ml of DMF with 7.2 ml of triethylamine as base analogously to Example 1, reaction time 5 h at 60 - 70 °C. The crude product is chromatographed over silica gel with methylene chloride / methanol 9:1. The fractions found to be uniform by TLC are combined. The oily residue is taken up in ethyl acetate and a little water, the aqueous phase is with conc. sodium hydroxide solution made alkaline. The aqueous phase is separated off, the organic phase is dried and the solvent is eliminated *in vacuo*. The residue is crystallised in acetone with methanesulphonic acid. 11 g of N-2-(3,5- N-[2-(3,5-bis-trifluoromethyl-phenyl)-ethyl]-2-[4-(2-hydroxy-1-hydroxymethylethylamino)-piperidin-1-yl]-N-methyl-2-phenylacetamide are obtained as a colourless methanesulphonate.

Particularly preferred NK-1 receptor antagonists of use in the present invention are compounds which are potent NK-1 receptor antagonists, i.e. compounds with an NK-1 receptor affinity of less than 10 nM, favourably less than 2 nM and preferably less than 1 nM.

The class of long acting NK-1 receptor antagonists of use in the present invention is identified using the following assay:

### ASSAY: Effect of NK1 receptor antagonist Ia, on SP-induced scratching in mice

### MATERIALS AND METHODS

### ANIMALS

Male ddY mice (Japan SLC, Ltd., Shizuoka, Japan) of 8-9 weeks of ages weighing 34-43 g, were used in the experiment. Mice were fasted 4 hours prior to the initiation of the experiment with free access to water.

### DRUGS

Epinastine and NK1 receptor antagonist of formula Ia as hydrochloride (Compound Ia)were provided by Boehringer Ingelheim Pharma K.G. (Germany). SP (lot. No. 80K5104) was supplied by Sigma Co., Ltd. (USA) and dissolved in physiological saline.

### METHODS

Before recording of behavior, the mice (two animals per one cage) were put into an acryl cage for at least 5 hours for acclimation. One hour after oral administration of vehicle (distilled water) or drugs (epinastine 10 mg/kg, Compound Ia 10, 30 mg/kg), SP was injected intradermally in a volume of 20 µL into the rostral back (around interscapular level). Immediately after the administration of SP, behaviors of mice was videotape registered using 8-mm video or digital video camera for 1 hour with any persons kept out the observation room (sound proof condition). Playing back of video served for counting scratching behavior. The mice generally showed several scratching by the hind paws for about 1sec and a series of these movements were counted as one beat of scratching.

### DATA ANALYSIS

Data were analyzed by One-way ANOVA *and post hoc* Tukey test; p<0.05 value was considered. Data presented as the mean ± S.E.

### RESULTS AND DISCUSSION

When mice were injected SP intradermally into the rostral back, the mice showed the scratching behavior and the count was 50.5 ± 12.1 beats/ 60 min. Compound Ia (30 mg/kg, p.o.) and epinastine (10 mg/kg, p.o.) showed the tendency to inhibit the scratching induced by SP, (21.1 ± 6.1 and 20.0 ± 8.4 beats / 60 min). Further effect of co-administration of epinastine and Compound Ia was not observed (Fig. 1).

In another experiment the mice were injected SP intradermally into the rostral back, and showed the scratching behaviour; the counts were 59.0 ± 6.0/ 60 min (Fig. 2a) and 51.6 ± 7.8 / 60 min (Fig. 2b). Compound Ia (30 mg/kg, p.o.) and epinastine (10 mg/kg, p.o.) reduced the scratching behaviour induced by SP to 29.9 ± 6.6 and 33.7 ± 4.0 beats / 60 min, respectively (Figs. 2a and 2b).

Furthermore, the dose dependency of the effect of Compound Ia on the SP-induced scratching behavior in ddY mice was examined in another experiment. The count of scratching behavior in vehicle treatment was 51.0 ± 12.9 beats/ 60 min, which was similar count to former experiment (50.5 ± 12.1 beats/ 60 min). Compound Ia (10 and 30 mg/kg, p.o.) inhibited the scratching induced by SP, and the effect was dose dependent (Fig. 3). In the experiments, the effects of the drugs was apparent..

In conclusion, Compound Ia showed the apparent inhibitory effect on scratching behaviour in ddY mice, and the effect was similar to the effect of epinastine, which is available drug for the treatment for itches of atopic dermatitis.

Comparable results are obtained with the compounds of formulae Ib to Id.

## Claims

1. Method for the treatment or prevention of atopic dermatitis, which comprises the administration of an effective amount of an NK-1 receptor antagonist to a patient in need of such treatment, wherein said NK1 receptor antagonist is effective in inhibiting the substance P (SP-)induced scratching in mice.

2. The method according to claim 1, wherein said NK-1 receptor antagonist, is administered orally.

3. The method according to claim 1 or 2, which comprises the administration of an effective amount of an NK-1 receptor antagonist, wherein said NK1 receptor antagonist is effective in reducing the SP-induced scratching in mice for at least 50 %.

4. The method according to any of the preceding Claims, which comprises the administration of an effective amount of an NK-1 receptor antagonist without concomitant therapy with corticosteroides.

5. The method according to any of the preceding Claims, wherein said NK-1 receptor antagonist is a phenyl-glycineamide having a structural element of formula (1) 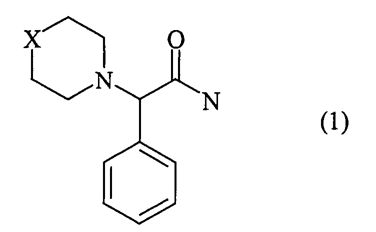 wherein X represents N or CH.

6. The method according to Claim 4, wherein said phenyl-glycineamide having a structural element of formula (1) consists essentially of the (S)-enantiomer.

7. The method according to any of the preceding claims, wherein said NK-1 receptor antagonist is a phenyl-glycineamide of formula (I) 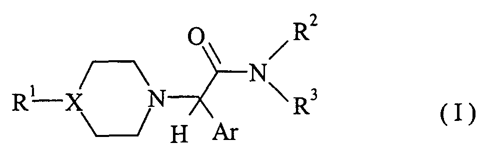 or a pharmaceutically acceptable salts thereof,
wherein
R¹ represents
(c) a C₃-C₈-cycloalkyl-C₁-C₆-alkyl group, in the event that X is a nitrogen atom; or
(d) an amino group of formula NR⁴R⁵, in the event that X is CH group;
R² represents phenyl-C₁-C₄-alkyl, wherein the phenyl group may be substituted by 1 to 3 substituent selected from the group consisting of halogen, hydroxy, C₁-C₄-alkyl, C₁-C₄-alkoxy, C₁-C₄-fluoroalkyl, C₁-C₄-fluoroalkoxy;
R³ represents hydrogen, C₁-C₄-alkyl, C₃-C₈-cycloalkyl, CH₂COOH, -CH₂C(O)NH₂, -OH or phenyl-C₁-C₄-alkyl;
R⁴ represents a 3-hydroxypropyl, 1,3-dihydroxyprop-2-yl or C₃-C₆-cycloalkylmethyl group;
R⁵ represents hydrogen, C₁-C₆-alkyl, ω-hydroxy-C₂-C₄-alkyl, 1,3-dihydroxyprop-2-yl or C₃-C₆-cycloalkylmethyl group; and
Ar represents an unsubstituted phenyl group or a phenyl group which is substituted by 1 to 5 substituents selected from the group consisting of halogen, hydroxy, C₁-C₄-alkyl, C₁-C₄-alkoxy, C₁-C₄-fluoroalkyl, C₁-C₄-fluoroalkoxy and -OCH₂O-.

8. The method according to claim 7, wherein -NR²R³ is a group of formula

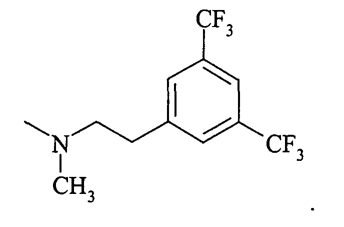

9. The method according to any of the preceding claims, wherein the said NK-1 receptor antagonist is selected from the formulae (Ia) to (Id):

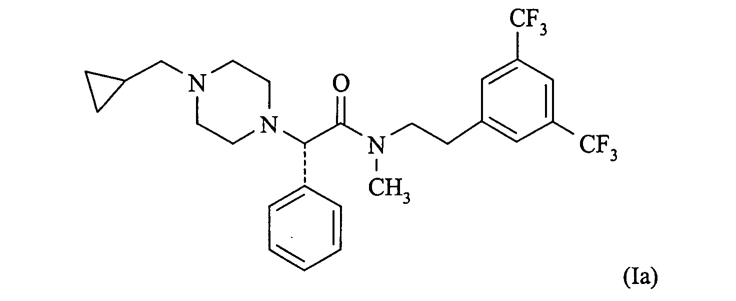

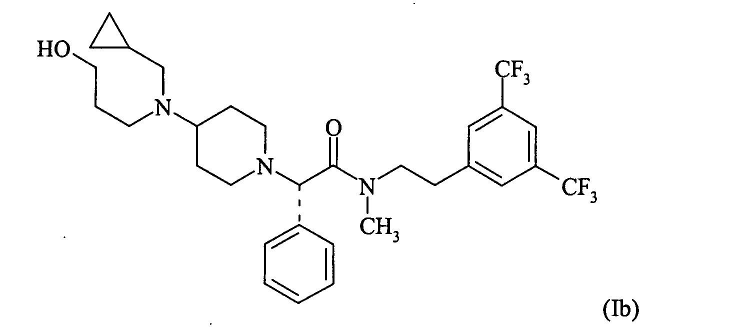

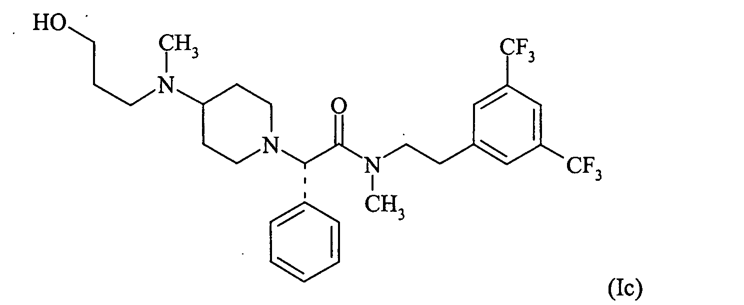

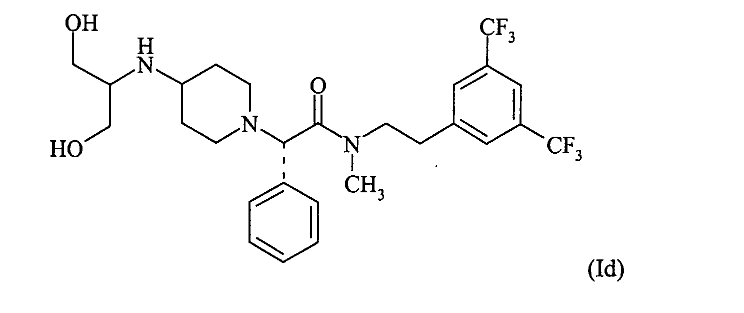

10. The method according to any of the preceding claims, which comprises the administration of 10 to 1500 mg per day of an NK-1 receptor antagonist.

11. A pharmaceutical composition for the treatment of atopic dermatitis, which comprises a long acting, NK-1 receptor antagonist, which is effective in inhibiting the SP-induced scratching in mice, together with a pharmaceutically acceptable carrier or excipient.

12. The use of an NK-1 receptor antagonist, which is effective in inhibiting the SP-induced scratching in mice for the preparation of a medication for the treatment or prevention of atopic dermatitis.

13. The use according to claim 12, wherein said NK1 receptor antagonist is effective in reducing the SP-induced scratching in mice for at least 50 %.
